# EUROPEAN PATENT APPLICATION

(11) **EP 4 438 791 A1**
(43) Date of publication of application: **02.10.2024**
(21) Application number: 22898821.8
(22) Date of filing: 04.10.2022
(51) Int. Cl.: D06F 39/00, D06F 73/02, D06F 58/26, D06F 33/34, D06F 33/54, D06F 34/14, A61L 2/07

(54) **CLOTHING TREATMENT APPARATUS PROVIDED WITH STEAM GENERATION DEVICE, AND METHOD FOR CONTROLLING CLOTHING TREATMENT APPARATUS**

(30) Priority: 25.11.2021 KR 20210164397
(71) Applicant: LG Electronics Inc., Yeongdeungpo-gu Seoul 07336 (KR)
(72) Inventor: KIM, Beomjun, Seoul 08592 (KR); CHA, Seungchul, Seoul 08592 (KR); KIM, Sunwoo, Seoul 08592 (KR); KIM, Youngho, Seoul 08592 (KR)
(74) Representative: Schott, Jakob Valentin
(86) International application number: PCT/KR2022/014879
(87) International publication number: WO 2023/096138

(57) **Abstract**

The present disclosure relates to a clothing treatment apparatus provided with a steam generation device, and a method for controlling the clothing treatment apparatus. The clothing treatment apparatus comprises: a cabinet, an accommodation part which is located inside the cabinet and accommodates an object; and a steam generation device which is located outside the object accommodation part and generates steam that is to be supplied to the accommodation part. The steam generation device includes: a housing that accommodates steam water and forms a heating space; a cover covering the housing; a first heater that heats the steam water from below a first water level; and a second heater that heats, from above the first water level, the steam water or steam heated by the first heater.

## Description

### TECHNICAL FIELD

The present disclosure relates to a clothing treatment apparatus including a steam generation device and a control method thereof, and more particularly to a clothing treatment apparatus configured through inclusion of heaters of different capacities in order to generate overheated steam by secondarily heating generated steam, thereby overheating the generated steam, and a control method of the clothing treatment apparatus.

### BACKGROUND ART

Generally, a clothing treatment apparatus may mean a home electronic appliance including a washing device configured to separate contaminants attached to clothing through actions of supplied wash water and detergent, a drying device configured to supply hot air to wet clothing, thereby drying the clothing, and a clothing management device configured to refresh clothing using hot air or steam in place of water-based washing.

Among recent clothing treatment apparatuses, there is a clothing treatment apparatus configured to enable sterilization, odor removal, wrinkle removal, etc. of clothing using a steam generation device. Conventional steam generation devices have a structure including a storage space configured to store water supplied from an exterior thereof, and a heater provided at an interior of the storage space such that the heater directly contacts the stored water.

Here, such a steam generation device is a device configured to generate steam and to supply the generated steam to an object such as clothing or tableware. In addition, steam performs functions as a heating source configured to heat an object and a moisture supply source configured to supply moisture to the object. Accordingly, such functions may be extended to not only washing machines, but also to various home electronic appliances.

That is, the steam generation device may be equipped in a washing machine to perform a function for generating high-temperature steam and supplying the steam during a washing operation for clothing, thereby enhancing a washing effect. In addition, the steam generation device may also be equipped in a washing machine having a drying function, that is, a clothing treatment apparatus such as a drying apparatus, a clothing management apparatus, etc., and, as such, may perform a refresh function for providing steam to remove wrinkles or odors from clothing, thereby providing feeling like new clothing.

In the following description, a washing machine will be mainly described as a representative example of a clothing treatment apparatus, but the clothing treatment apparatus may be applicable to other washing machines, drying apparatuses, clothing management apparatuses, and home electronic appliances so long as the apparatuses or appliances are not exclusive or incompatible therefrom.

Hereinafter, a steam generation device for a conventional washing machine will be described with reference to the accompanying drawings.

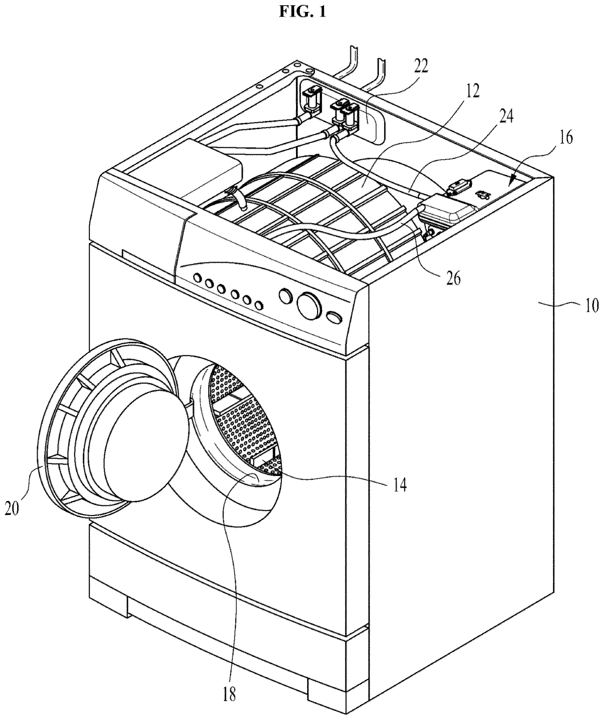

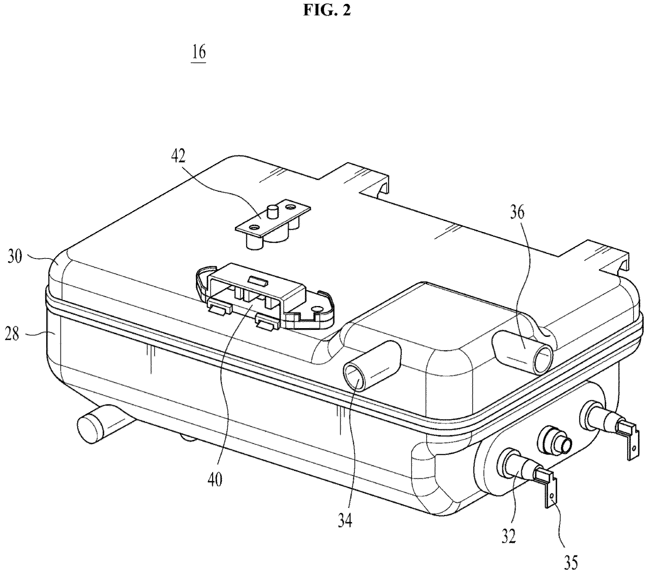

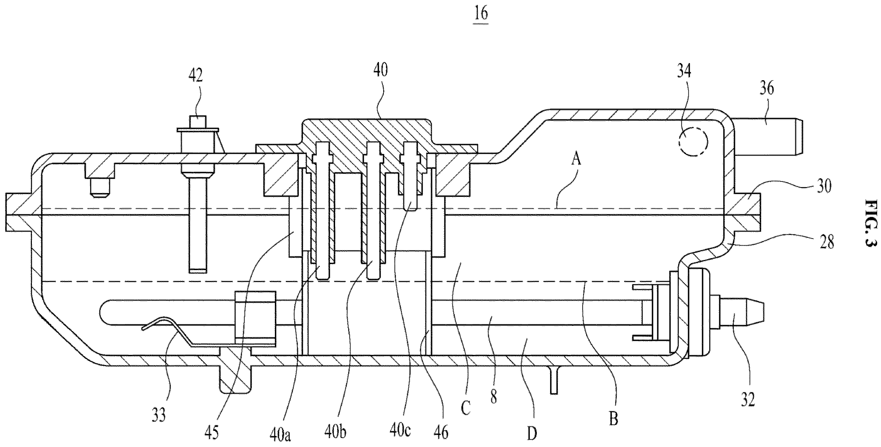

FIG. 1 is a perspective view briefly showing a conventional washing machine. FIG. 2 is a perspective view showing a conventional steam generation device. FIG. 3 is a sectional view showing an inner structure of the steam generation device shown in FIG. 2.

As shown in FIG. 1, the washing machine equipped with the conventional steam generation device is configured through inclusion of a case 10 configured to form an outer appearance, a cylindrical tub 12 horizontally supported in the case 10 and configured to store wash water, a drum 14 rotatably installed in the tub 12, and a steam generation device 16 configured to supply steam to an interior of the drum 14.

Here, the drum is an accommodation part configured to accommodate an object and, as such, accommodates clothing, etc., that is, objects to be washed. In addition, in a drying device, clothing, etc., that is, objects to be dried, are accommodated. Similarly, for refresh, dried clothing may be accommodated in an object accommodation part. Accordingly, the above-mentioned accommodation part may be extended to various configurations in accordance with a shape thereof, a kind of object, and a function and a shape of a home electronic appliance. That is, the accommodation part may be diversely extended to an accommodation part in which tableware is accommodated, an accommodation part in which clothing to be refreshed is accommodated, an inner tub of a top load washing machine, etc.

A feeding port 18, which communicates with the interior of the drum 14, is formed at a front surface of the case 10 in order to load or unload clothing. A door 20 is forwardly pivotably installed at the case 10 in order to open or close the feeding port 18.

Meanwhile, a water supply valve 22 and a water supply hose 24 are provided at one side of the case 10 in order to supply water to the steam generation device 16.

In addition, a steam supply tube 26, which functions as a passage for guiding and injecting steam generated in the steam generation device 16 to the interior of the drum 14, is connected to one side of the steam generation device 16.

Hereinafter, the steam generation device 16 will be described in more detail with reference to FIGs. 2 and 3.

The steam generation device 16 is configured through inclusion of a lower case 28 configured to form a space for storing water, an upper case 30 coupled to an upper portion of the lower case 28, and a heater 32 configured to heat water stored in an interior of the steam generation device 16.

The upper case 30 is provided with a water supply port 34 configured to receive water supplied from the water supply hose 24, and a steam discharge port 36 configured to discharge steam generated in the steam generation device 16 into the steam supply tube 26.

Meanwhile, the heater 32 is installed at a lower portion of the lower case 28 in parallel to a bottom surface of the lower case 28. When water is supplied to the steam generation device 16, the heater 32 operates to heat the water in a state of being completely submerged in the water.

A mounting structure of the heater 32 will be described hereinafter in more detail.

As shown in FIG. 3, the heater 32 is longitudinally inserted into an interior of the cases 28 and 30 formed to have a rectangular shape, through one side surface of the cases 28 and 30 having a smaller area among side surfaces of the cases 28 and 30, in parallel to the lower portion of the lower case 28. Of course, the side surface, through which the heater 32 is inserted, may be sealed in order to prevent water leakage, etc. therefrom, and electric power may be supplied to the heater 32 through a terminal 35.

Meanwhile, a bracket 33 is provided at the bottom surface of the lower case 28. The heater 32 is inserted into and fixed to the bracket 33. Accordingly, the heater 32 is fixed, at one side thereof, to the bracket 33 while being fixed, at the other side thereof, to the one side surface of the cases 28 and 30.

A water level sensor 40 configured to sense a water level of water stored in the steam generation device 16 is provided at one side of the upper case 30. A temperature sensor 42 configured to measure a temperature of water heated by the heater 32 and a temperature of steam generated by the heater 32 is provided at a central portion of the upper case 30.

The water level sensor 40 may be configured through inclusion of a high water level electrode rod 40c, a low water level electrode rod 40b, and a common electrode rod 40a in order to sense a high water level and a low water level. In addition, barrier walls 45 and 46 surrounding the water level sensor 40 may be provided. The barrier walls 45 and 46 perform a function for stably maintaining a sensed water level, thereby reducing errors of water level sensing.

The conventional steam generation device configured as mentioned above operates as follows.

First, when a washing process of the washing machine starts, water is introduced into the interior of the steam generation device 16 through the water supply port 34.

The water introduced into the interior of the steam generation device 16 is heated by the heater 32 and, as such, is changed into steam. The steam is introduced, through the steam discharge port 36, into the interior of the drum 14 in which clothing is accommodated and, as such, performs wetting and soaking actions, thereby enhancing washing efficiency.

In this case, the steam discharged through the steam discharge port 36 is high-temperature steam. Of course, when a discharge valve configured to be opened or closed by a pressure of steam is provided at an upstream or downstream side of the steam discharge port 36, the steam discharged through the steam discharge portion 36 may be high-temperature and high-pressure steam. However, in either case, steam may be supplied to the drum by a pressure thereof.

Meanwhile, after completion of the above-mentioned wetting and soaking actions for the clothing, operation of the steam generation device 16 is stopped, and a series of main washing processes is performed and, as such, washing of clothing is completed.

In the conventional steam generation device as mentioned above, the heater operates in a state in which the interior of the storage space has been filled with a predetermined amount of water. For this reason, the conventional steam generation device has a structure capable of supplying steam only when the heater operates in a state in which the interior of the storage space is completely filled with water, and the stored water is then boiled in accordance with the operation of the heater.

For this reason, the structure of the conventional steam generation device takes a lot of time to generate steam and adjusting a temperature of steam discharged from the steam generation device is difficult.

In addition, since the conventional steam generation device is provided with only the heater, which has a fixed capacity, for generation of steam, it is difficult to adjust a generation amount of steam in accordance with an amount of clothing. When the amount of clothing is great, there is a problem in that a lot of time is taken to generate an appropriate amount of steam.

Furthermore, the conventional steam generation device uses only steam generated through boiling of water heated by the heater. In this regard, the temperature of the steam is relatively low. For this reason, when the supply distance of steam (that is, the length of the steam supply tube) is lengthened, there is a problem in that the temperature of steam is lowered in accordance with the steam supply distance and, as such, supply of steam is inefficiently carried out.

In addition, the structure of the conventional steam generation device inevitably generates unsaturated steam due to a structural limitation in which steam is generated through heating of water. In recent years, however, utilization of overheated steam of 100°C or more has been proposed for rapid heating of clothing and wash water.

Furthermore, since the structure of the conventional steam generation device inevitably generates unsaturated steam due to a structural limitation in which steam is generated through heating of water, utilization of overheated steam of 100°C or more has recently been proposed in order to achieve an enhancement in sterilization ability which has also recently been proposed.

### DISCLOSURE

### TECHNICAL TASK

One technical task of the present disclosure devised to solve the problem lies in a clothing treatment apparatus including a steam generation device capable of selectively generating steam of a required capacity by adjusting a steam generation amount in accordance with the required steam capacity upon clothing treatment, and a control method of the clothing treatment apparatus.

Another technical task of the present disclosure devised to solve the problem lies in a steam generation device capable of more rapidly generating steam through preheating and heating processes in accordance with supply of normal steam water, and a clothing treatment apparatus using the steam generation device.

Another technical task of the present disclosure devised to solve the problem lies in a clothing treatment apparatus including a steam generation device capable of supplying steam of a small capacity or a large capacity in accordance with supply of normal steam water, and a control method of the clothing treatment apparatus.

Another technical task of the present disclosure devised to solve the problem lies in a clothing treatment apparatus including a steam generation device capable of generating and supplying overheated steam having a higher temperature than a normal steam temperature, and a control method of the clothing treatment apparatus.

Another technical task of the present disclosure devised to solve the problem lies in a clothing treatment apparatus including a steam generation device capable of generating overheated steam by secondarily heating generated steam, and a control method of the clothing treatment apparatus.

Another technical task of the present disclosure devised to solve the problem lies in a clothing treatment apparatus including a steam generation device capable of supplying high-temperature steam even when a movement distance of steam is lengthened, by generating overheated steam having a higher temperature than a normal steam temperature, and a control method of the clothing treatment apparatus.

Another technical task of the present disclosure devised to solve the problem lies in a clothing treatment apparatus including a steam generation device capable of generating overheated steam by generating steam using a small amount of water, and secondarily heating the generated steam, and a control method of the clothing treatment apparatus.

The technical tasks of the present disclosure are not limited to the above-described technical tasks and other technical tasks which are not described herein may be derived by those skilled in the art from the following description of the present disclosure.

### TECHNICAL SOLUTIONS

In one technical aspect of the present disclosure for accomplishing the technical tasks, provided is a clothing treatment apparatus including a cabinet, an accommodation part disposed inside the cabinet and configured to accommodate an object, and a steam generation device disposed outside the object accommodation part and configured to generate steam to be supplied to the object accommodation part, wherein the steam generation device includes: a housing configured to receive steam water and to form a heating space; a cover configured to cover the heating space; a first heater configured to preheat the steam water at a first water level or to heat the steam water, thereby generating steam; and a second heater configured to heat steam water at a second water level higher than the first water level, thereby generating steam, or to heat the steam generated at the first water level in a space between the first water level and the second water level, thereby generating overheated steam.

The steam generation device may further include a water level sensing unit configured to sense the first water level and the second water level of steam water supplied to the heating space.

The water level sensing unit may include a first water level electrode configured to sense the first water level, and a second water level electrode configured to sense the second water level.

The first heater may preheat and heat the steam water of the first water level in accordance with water level sensing of the first water level electrode and the second heater may heat the steam water of the second water level in accordance with water level sensing of the second water level electrode, thereby generating steam.

The steam generation device may further include a temperature sensor configured to sense an internal temperature of the heating space, thereby sensing generation of steam. The second heater may heat steam over the first water level in accordance with steam sensing of the temperature sensor, thereby generating overheated steam.

The steam generation device may further include a lower barrier wall configured to form a movement path of the steam generated at the first water level, and an upper barrier wall disposed over the second heater and configured to form a movement path of the steam together with the lower barrier wall.

The second heater may be disposed between the lower barrier wall and the upper barrier wall and may heat the steam moving between the lower barrier wall and the upper barrier wall, thereby generating overheated steam.

Each of the lower barrier wall and the upper barrier wall may be formed with a plurality of through holes configured to diffuse the steam and the overheated steam.

The lower barrier wall may be provided at the housing such that one side thereof is opened, and the upper barrier wall may be provided at the housing such that another side thereof is opened, to form the movement path of the steam generated at the first water level.

In another technical aspect of the present disclosure for accomplishing the technical tasks, provided is a clothing treatment apparatus including a cabinet, an accommodation part disposed inside the cabinet and configured to accommodate an object, and a steam generation device disposed outside the object accommodation part and configured to generate steam to be supplied to the object accommodation part, wherein the steam generation device includes: a housing configured to receive steam water and to form a heating space; a cover configured to cover the heating space; a water level sensing unit configured to sense a first water level and a second water level of steam water supplied to the heating space, the second water level being higher than the first water level; a first heater configured to heat steam water below the first water level; and a second heater configured to heat steam generated over the first water level through heating by the first heater, thereby generating overheated steam, or to heat the steam water supplied up to the second water level, thereby generating steam.

The water level sensing unit may include a first water level electrode configured to sense the first water level, and a second water level electrode configured to sense the second water level.

The first heater may preheat and heat the steam water of the first water level in accordance with water level sensing of the first water level electrode and the second heater may heat the steam water of the second water level in accordance with water level sensing of the second water level electrode, thereby generating steam.

The steam generation device may further include a temperature sensor configured to sense an internal temperature of the heating space, thereby sensing generation of steam. The second heater may heat steam over the first water level in accordance with steam sensing of the temperature sensor, thereby generating overheated steam.

The steam generation device may further include a lower barrier wall configured to form a movement path of the steam generated at the first water level, and an upper barrier wall disposed over the second heater and configured to form a movement path of the steam together with the lower barrier wall.

The second heater may be disposed between the lower barrier wall and the upper barrier wall and may heat the steam moving between the lower barrier wall and the upper barrier wall, thereby generating overheated steam.

Each of the lower barrier wall and the upper barrier wall may be formed with a plurality of through holes configured to diffuse the steam and the overheated steam.

The lower barrier wall may be provided at the housing such that one side thereof is opened, and the upper barrier wall may be provided at the housing such that another side thereof is opened, to form the movement path of the steam generated at the first water level.

The second heater may heat steam moving between the lower barrier wall and the upper barrier wall, thereby generating overheated steam.

In another technical aspect of the present disclosure for accomplishing the technical tasks, provided is a clothing treatment apparatus including a cabinet, an accommodation part disposed inside the cabinet and configured to accommodate an object, and a steam generation device disposed outside the object accommodation part and configured to generate steam to be supplied to the object accommodation part, wherein: the steam generation device includes: a box-shaped housing configured to receive steam water supplied thereto; a cover configured to cover the heating space; a first heater disposed under the housing; a lower barrier wall disposed over the first heater; a second heater disposed over the lower barrier wall; and an upper barrier wall disposed over the second heater; and steam generated through heating by the first heater is moved to a space between the lower barrier wall and the upper barrier wall and is then changed into overheated steam by the second heater.

The lower barrier wall may guide the steam generated by the first heater to move between the lower barrier wall and the upper barrier wall, and the lower barrier wall and the upper barrier wall may form a steam heating space heated by the second heater.

Each of the lower barrier wall and the upper barrier wall may be formed with a plurality of through holes configured to diffuse the steam and the overheated steam.

The lower barrier wall may be provided at the housing such that one side thereof is opened, and the upper barrier wall may be provided at the housing such that another side thereof is opened, to form a movement path of the steam generated at the first water level.

In another technical aspect of the present disclosure for accomplishing the technical tasks, provided is a clothing treatment apparatus including a cabinet, an accommodation part disposed inside the cabinet and configured to accommodate an object, and a steam generation device disposed outside the object accommodation part and configured to generate steam to be supplied to the object accommodation part, wherein: the steam generation device includes: a housing configured to receive steam water and to form a heating space; a cover configured to cover the heating space; a water level sensing unit configured to sense a first water level and a second water level of steam water supplied to the heating space, the second water level being higher than the first water level; a first heater disposed below the first water level; a second heater disposed between the first water level and the second water level; a lower barrier wall disposed between the first water level and the second heater; and an upper barrier wall disposed above the second water level and configured to form a steam flow path together with the lower barrier wall; and the second heater heats steam generated through heating by the first heater when the steam water is disposed at the first water level, and heats the steam water together with the first heater when the steam water is disposed at the second water level, thereby generating steam.

The water level sensing unit may include a first water level electrode configured to sense the first water level, and a second water level electrode configured to sense the second water level. The first heater may preheat and heat the steam water of the first water level in accordance with water level sensing of the first water level electrode.

The steam generation device may further include a temperature sensor configured to sense an internal temperature of the heating space, thereby sensing generation of steam. The second heater may heat steam over the first water level in accordance with steam sensing of the temperature sensor, thereby generating overheated steam.

In another technical aspect of the present disclosure for accomplishing the technical tasks, provided is a control method of a clothing treatment apparatus including a steam generation device including a water level sensing unit configured to sense a first water level and a second water level of steam water supplied to a heating space, the second water level being higher than the first water level, a first heater disposed below the first water level, and a second heater disposed between the first water level and the second water level, the control method including: water supply of supplying steam water to the steam generation device; first water level sensing of sensing the first water level of the supplied steam water; preheating of preheating the steam water of the first water level by the first heater; second water level sensing of sensing the second water level higher than the first water level; and heating of heating the steam water of the second water level by the second heater in accordance with the sensing of the second water level, thereby generating steam.

The control method may further include second water level maintenance sensing of determining whether or not a water level of the steam water is maintained at the second water level, after the heating.

The control method may further include additional water supply of supplying additional steam water when the water level of the steam water is lowered below the second water level in the second water level maintenance sensing.

In another technical aspect of the present disclosure for accomplishing the technical tasks, provided is a control method of a clothing treatment apparatus including a steam generation device including a water level sensing unit configured to sense a first water level and a second water level of steam water supplied to a heating space, the second water level being higher than the first water level, a first heater disposed below the first water level, and a second heater disposed between the first water level and the second water level, the control method including: water supply of supplying steam water to the steam generation device; first water level sensing of sensing the first water level of the supplied steam water; heating of heating the steam water of the first water level by the first heater, thereby generating steam; and overheated steam generation of heating the steam generated at the first water level by the second heater, thereby generating overheated steam.

The control method may further include steam sensing of measuring a temperature of the heating space after the heating, thereby sensing generation of the steam.

The control method may further include first water level maintenance sensing of determining whether or not a water level of the steam water is maintained at the first water level, after the heating.

The control method may further include additional water supply of supplying additional steam water when the water level of the steam water is lowered below the second water level in the first water level maintenance sensing.

### ADVANTAGEOUS EFFECTS

The clothing treatment apparatus including the steam generation device and the control method of the clothing treatment apparatus according to the present disclosure have an effect of selectively generating a desired amount of steam by adjusting a steam generation amount in accordance with a steam capacity required in treatment of clothing.

In addition, the clothing treatment apparatus including the steam generation device and the control method of the clothing treatment apparatus according to the present disclosure have an effect of generating steam within a shorter time through preheating and heating procedures in accordance with supply of normal steam water.

In addition, the clothing treatment apparatus including the steam generation device and the control method of the clothing treatment apparatus according to the present disclosure have an effect of supplying a small amount of steam or a large amount of steam in accordance with supply of normal steam water.

In addition, the clothing treatment apparatus including the steam generation device and the control method of the clothing treatment apparatus according to the present disclosure have an effect of generating and supplying overheated steam having a higher temperature than a normal steam temperature.

In addition, the clothing treatment apparatus including the steam generation device and the control method of the clothing treatment apparatus according to the present disclosure have an effect of generating overheated steam by secondarily heating generated steam.

In addition, the clothing treatment apparatus including the steam generation device and the control method of the clothing treatment apparatus according to the present disclosure have an effect of supplying steam of a high temperature even when a movement distance of steam is lengthened, by generating overheated steam having a higher temperature than a normal steam temperature.

Furthermore, the clothing treatment apparatus including the steam generation device and the control method of the clothing treatment apparatus according to the present disclosure have an effect of generating overheated steam by generating steam using a small amount of water, and secondarily heating the generated steam.

Effects attainable in the present disclosure are not limited to the above-described effects, and other effects of the present disclosure not yet described will be more clearly understood by those skilled in the art from the description of the claims.

### DESCRIPTION OF DRAWINGS

FIG. 1 is a perspective view briefly showing a conventional washing machine.
FIG. 2 is a perspective view showing a conventional steam generation device.
FIG. 3 is a sectional view showing an inner structure of the steam generation device shown in FIG. 2.
FIG. 4 is a perspective view showing a steam generation device according to an embodiment of the present disclosure.
FIG. 5 is an exploded perspective view showing the steam generation device according to the embodiment of the present disclosure.
FIG. 6 is a sectional view showing an inner structure of the steam generation device according to the embodiment of the present disclosure.
FIG. 7 is a flowchart showing an operation procedure of a clothing treatment apparatus including the steam generation device according to the embodiment of the present disclosure.
FIG. 8 is a flowchart showing a steam generation procedure of the steam generation device according to an embodiment of the present disclosure.
FIG. 9 is a flowchart showing a procedure for generating a large amount of steam in the steam generation device in accordance with an embodiment of the present disclosure.
FIG. 10 is a flowchart showing a procedure for generating overheated steam in the steam generation device in accordance with an embodiment of the present disclosure.

### BEST MODE FOR DISCLOSURE

Hereinafter, a clothing treatment apparatus including a steam generation device according to an embodiment of the present disclosure and a control method of the clothing treatment apparatus will be described in detail with reference to the accompanying drawings.

In describing the present disclosure, names of constituent elements are defined taking into consideration functions thereof in the present disclosure. Accordingly, the names should not be understood as meanings limiting technical constituent elements of the present disclosure. In addition, names defined for constituent elements may also be referred to as other names in the technical field to which the present disclosure pertains.

First, a steam generation device according to an embodiment of the present disclosure and a clothing treatment apparatus using the steam generation device will be described in detail with reference to the accompanying drawings.

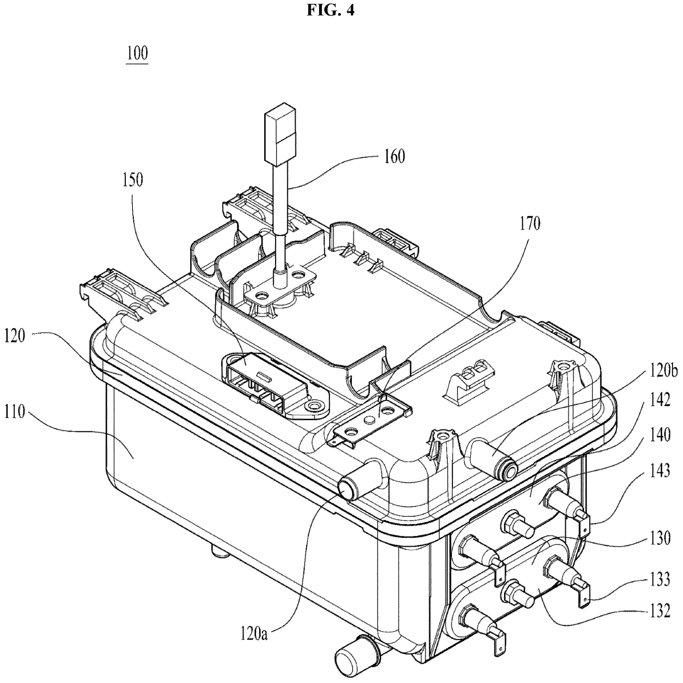

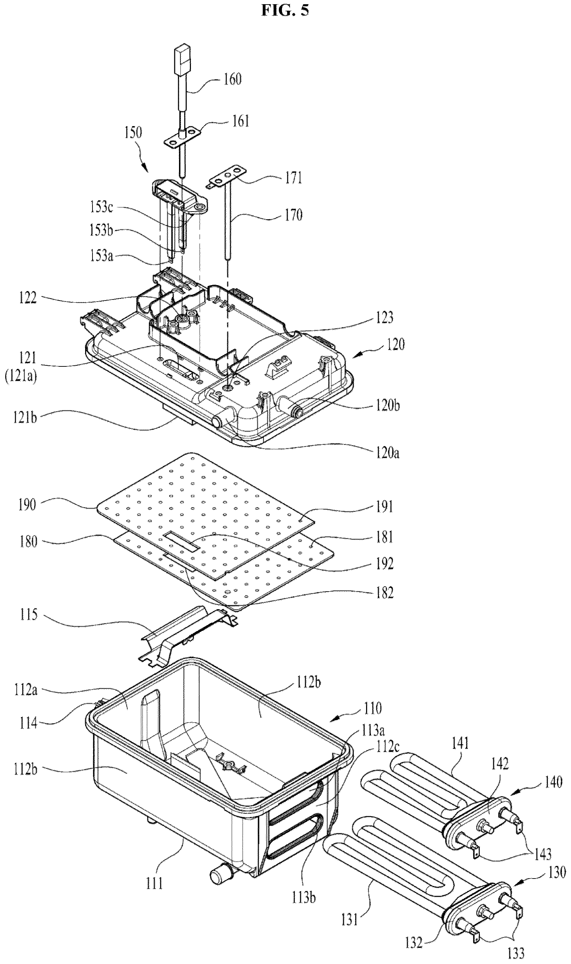

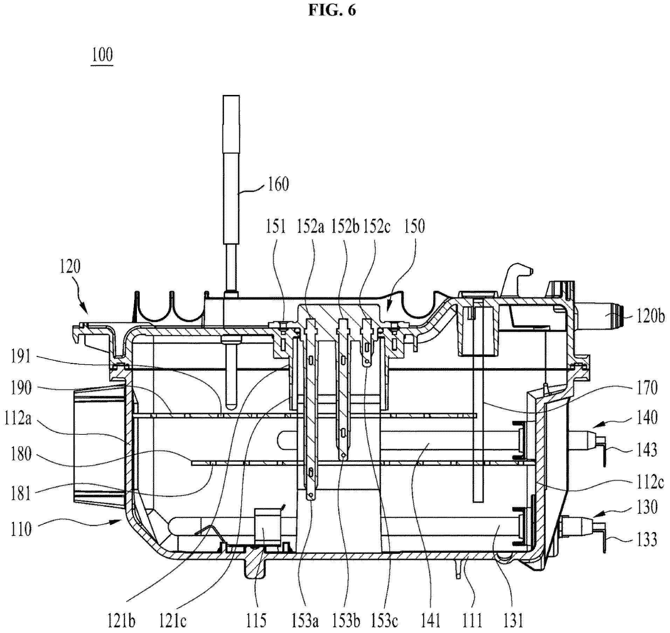

FIG. 4 is a perspective view showing a steam generation device according to an embodiment of the present disclosure. FIG. 5 is an exploded perspective view showing the steam generation device according to the embodiment of the present disclosure. FIG. 6 is a sectional view showing an inner structure of the steam generation device according to the embodiment of the present disclosure.

As shown in FIGs. 4 and 5, the steam generation device 100 according to the embodiment of the present disclosure may include a box-shaped housing 110 configured to form a storage space in which steam water is temporarily stored, a first heater 130 coupled to the housing 110 and configured to heat the steam water stored in the housing 110, thereby generating steam (unsaturated vapor or wet vapor)(referred to as "steam" hereinafter), a second heater 140 coupled to the housing 110 in a state of being upwardly spaced apart from the first heater 130 and configured to heat the steam water stored in the housing 110 or the steam generated by the first heater 130, thereby generating overheated steam (overheated vapor) (referred to as "overheated steam" hereinafter), a cover 120 configured to cover an opened surface of the housing 110, a water level sensing unit 150 provided at the cover 120 and configured to determine a water level of the steam water stored in the housing 110, and a temperature sensor 160 configured to sense a temperature of steam generated by the first heater 130 and the second heater 140.

Meanwhile, the steam generated by the first heater 130 is a kind of vapor having a most general form and typically includes moisture of water molecules not vaporized. Typically, the steam contains 3 to 5% of moisture. Such steam has a temperature of about 100°C, and may be used for heating of clothing or wash water and sterilization at a relatively low temperature.

In addition, the overheated steam generated by the second heater 140 has a temperature higher than that of steam (at least 100°C or more) and is generated when a vaporization speed and a liquefaction speed are equal. Such overheated steam may enable rapid and uniform heating by virtue of a high coefficient of heat transfer according to latent heat thereof and may be used for rapid heating of clothing or wash water or sterilization at a relatively high temperature.

In this case, a lower barrier wall 180 and an upper barrier wall 190 may be further formed under and over the second heater 140, respectively, such that the lower barrier wall 180 and the upper barrier wall 190 are spaced apart from the second heater 140 by a predetermined distance, to form a space in which the steam generated by the first heater 130 may stay temporarily.

The housing 110 may be constituted by a base 111 configured to form a bottom surface of the storage space, and side walls 112a, 112b, 112c, and 112d configured to form side surfaces of the storage space. The inner storage space may be formed by the base 111 and the side walls 112a, 112b, 112c, and 112d.

In addition, the housing 110 may be formed to have a rectangular shape. That is, the housing 110 may be constituted by the base 111, which has a rectangular shape, and the side walls. Since the base 111 has a rectangular shape, the base 111 may be defined as having a length in a longitudinal direction greater than a length in a width direction. Accordingly, the housing 110 may be formed to have a box shape in which the side walls 112b and 112d in the longitudinal direction and the side walls 112a and 112c in the width direction extend upwards from the base 111, and a top of the housing 110 is opened.

Meanwhile, the storage space in which water is accommodated is formed by the base 111 and the side walls 112a, 112b, 112c, and 112d. Of course, the housing 110 may accommodate steam at an upper portion thereof or the entire portion thereof as water therein is heated. Accordingly, the housing 110 may form a heating space configured to heat steam water.

In this case, among the side walls 112a, 112b, 112c, and 112d, the side wall 12c formed at a rear side may be formed with a lower heater mounting hole 113b and an upper heater mounting hole 113a for coupling of the first heat 130 and the second heater 140 thereto.

Meanwhile, the first heater 130 includes a first heater bracket 132 fixed to the lower heater mounting hole 113b, a first heater coil 131 extending from the first heater bracket 132 to an inside of the housing 110, and a first heater terminal 143 provided at the first heater bracket 132 outside the housing 110. A power source is connected to the first heater terminal 143.

In addition, the second heater 140 includes a second heater bracket 142 fixed to the upper heater mounting hole 113a, a second heater coil 141 extending from the second heater bracket 142 to the inside of the housing 110, and a second heater terminal 143 provided at the second heater bracket 142 outside the housing 110. The power source is connected to the second heater terminal 143.

In this case, the first heater 130 and the second heater 140 may be inserted into and then fixedly coupled to the lower heater mounting hole 113b and the upper heater mounting hole 113a of the housing 110 through separate manufacturing processes, respectively. Otherwise, in manufacture of the housing 110, the housing 110 may be injection-molded using a heat-resistant synthetic resin material, and the first heater bracket 132 of the first heater 130 and the second heater bracket 142 of the second heater 140 may be formed to be insert injection-molded at the lower heater mounting hole 113b and the upper heater mounting hole 113a, respectively.

Meanwhile, it is preferred that the first heater 130 and the second heater 140 may be formed to have different output powers, respectively. In detail, the second heater 140 may be configured to have a lower output power than that of the first heater 130. For example, when it is assumed that the first heater 130 has an output power of 1,000 W, the second heater 140 may be configured to have an output power of 600 W. Typically, in a clothing treatment apparatus, a maximum current in operation of the clothing treatment apparatus is limited. When current exceeding the maximum current is generated, the current load in the home may be overloaded. To this end, output powers of the first heater 130 and the second heater 140 may be varied taking into consideration the current load in the home.

In addition, the first heater 130 may be configured to always heat steam water, and the second heater 140 may be configured to selectively heat steam water or steam. Accordingly, the first heater coil 131 of the first heater 130 and the second heater coil 141 of the second heater 140 may be formed of different materials in order to heat different objects, respectively.

Meanwhile, the first heater 130 and the second heater 140 as described above may operate simultaneously or individually, and the second heater 140 may be controlled to re-heat wash water heated by the first heater 130 or to re-heat steam generated from steam water heated by the first heater 130, thereby generating overheated steam. Control procedures of the first heater 130 and the second heater 140 will be described in detail upon describing control of the steam generation device 100.

Meanwhile, an upper support rib (not shown) and a lower support rib (not shown) configured to support the lower barrier wall 180 and the upper barrier wall 190 may be formed at inner peripheral surfaces of the side walls 112a, 112b, 112c, and 112d of the housing 110 to protrude while being spaced apart from each other by a predetermined distance in an upward/downward direction.

In this case, the upper support rib and the lower support rib support the upper barrier wall 190 and the lower barrier wall 180, respectively. The first heater coil 131 of the first heater 130 is disposed under the lower barrier wall 180 supported by the lower support rib to extend along the lower barrier wall 180. The second heater coil 141 of the second heater 140 is disposed under the upper barrier wall 190 supported by the upper support rib to extend along the upper barrier wall 190. That is, the second heater coil 141 may be formed to extend in a space between the lower barrier wall 180 and the upper barrier wall 190.

Meanwhile, the heating space of the housing 110 may be divided into different spaces in accordance with objects heated in the heating space. That is, in the present disclosure, as described above, the first heater 130 configured to heat steam water and the second heater 140 configured to heat steam generated from the steam water heated by the first heater 130 are provided.

In this case, the first heater 130 may be disposed at a lower portion of the heating space and may heat steam water stored in the heating space, whereas the second heater 140 may heat steam generated over the steam water stored in the heating space.

Accordingly, the space in which steam water is heated by the first heater 130 may be referred to as a steam water heating space, and the space in which steam is heated by the second heater 140 may be referred to as a steam heating space.

Meanwhile, a plurality of through holes 181 and 191, through which steam water and steam pass, may be formed at the lower barrier wall 180 and the upper barrier wall 190. In this case, the through holes 181 formed at the lower barrier wall 180 may be configured to allow steam or steam water generated by the first heater 130 to pass therethrough. In addition, the plurality of through holes 191 formed at the upper barrier wall 190 may be configured to allow steam water or steam heated by the second heater 140 to pass therethrough.

In addition, the lower barrier wall 180 may be disposed between the first heater 130 and the second heater 140. The lower barrier wall 180 and the upper barrier wall 190 as described above may function to partition heating spaces of steam water or steam heated by the first heater 130 and the second heater 140 from each other.

Meanwhile, the lower barrier wall 180 and the upper barrier wall 190 may be configured to open one side and the other side of the heating space, respectively. That is, the lower barrier wall 180 may be formed to be spaced apart from the side wall 112a of the housing 110 by a predetermined distance, and the upper barrier wall 190 may be formed to be spaced apart from the side wall 112c of the housing 110 by a predetermined distance.

Accordingly, steam generated from steam water heated by the first heater 130 may move between the lower barrier wall 180 and the upper barrier wall 190 through a space between the lower barrier wall 180 and the side wall 112a. The steam moved between the lower barrier wall 180 and the upper barrier wall 190 may be heated by the second heater 140 and, as such, may be changed into overheated steam.

In this case, the plurality of through holes 181 and 191 formed at the lower barrier wall 180 and the upper barrier wall 190 may not only allow movement of steam water therethrough, but also may allow steam generated by the first heater 130 to be diffused into the heating space therethrough.

That is, steam water, which is heated by the first heater 130, may be heated by the first heater 130 at a water level below the lower barrier wall 180, thereby generating steam, and the generated steam may be diffused over the lower barrier wall 180 while passing through the through holes 181 of the lower barrier wall 180 and, as such, may be diffused in the space between the lower barrier wall 180 and the upper barrier wall 190.

In addition, the steam moved to the space between the lower barrier wall 180 and the upper barrier wall 190 is re-heated by the second heater 140 and, as such, is changed into overheated steam. In addition, the overheated steam heated between the lower barrier wall 180 and the upper barrier wall 190 may be diffused while passing through the plurality of through holes 191 formed at the upper barrier wall 190 and may then be discharged through a steam discharge port 120b formed at the cover 120.

Meanwhile, a first heater fixing bracket 115 configured to support the first heater coil 131 of the first heater 130 may be provided at an upper surface of the base 111 of the housing 110. Although the housing 110 may be manufactured through injection molding using a heat-resistant synthetic resin, the housing 110 may be deformed when the first heater 130 disposed at the lower portion of the heating space of the housing 110 directly contacts the housing 110. To this end, the first heater fixing bracket 115 configured to support the first heater coil 131 of the first heater 130 may be provided to enable the base 111 of the housing 110 and the first heater 130 to maintain a predetermined distance therebetween.

The cover 120 may be formed to cover an opened top surface of the housing 110, and may be injection-molded using the same heat-resistant synthetic resin material as that of the housing 110. A lower peripheral surface of the cover 120 may be formed to have a shape corresponding to that of the opened top surface of the housing 110, and may be integrally coupled to the housing 110 through a coupling method such as ultrasonic welding.

Meanwhile, a steam water supply port 120a configured to supply steam water to the heating space of the housing 110 is formed at one side of the cover 120. The steam water supply port 120a may receive steam water through a water supply part (not shown) of the clothing treatment apparatus. In addition, a separate control valve or a pressure reducing valve may be provide at a passage configured to interconnect the water supply part and the steam water supply port 120a, to control supply of steam water. In addition, a flow meter configured to sense a flow rate of steam water supplied to the housing 110 may be further provided.

In addition, the steam discharge port 120b, which supplies steam generated in the heating space of the housing 110, is formed at the other side of the cover 120. The steam discharge port 120b may supply steam to an accommodation part, such as a tub (not shown) or a drum (not shown), configured to receive clothing, using a hose (not shown) configured to form a steam passage.

Meanwhile, the water level sensing unit 150, the temperature sensor 160, and a common electrode 170 may be provided at an upper surface of the cover 120 while extending to an inside of the heating space of the housing 110 through the cover 120. For this configuration, a water level sensor mounting part 121 to which the water level sensing unit 150 is mounted, a temperature sensor mounting part 122 to which the temperature sensor 160 is mounted, and a common electrode mounting part 123 to which the common electrode 170 is mounted may be provided at an inside of the cover 120.

In this case, the water level sensor mounting part 121 may be formed with a through hole 121a configured to allow extension of the water level sensing unit 150 therethrough, and a water level sensor receiving portion 121b disposed at a lower portion of the cover 120 adjacent to the through hole 121a while extending toward the heating space by a predetermined distance. In this case, the water level sensor receiving portion 121b functions to block flow of steam water adjacent to electrodes 153a, 153b, and 153c of the water level sensing unit 150, which will be described later, thereby preventing malfunction of the water level sensing unit 150.

In this case, openings 121c may be formed at opposite sides of the water level sensor receiving portion 121b. The openings 121c may be formed to have a communication structure enabling introduction of steam water into an interior of the water level sensor receiving portion 121b or outward movement of air present in the interior of the water level sensor receiving portion 121b.

Meanwhile, the water level sensing unit 150 is configured through inclusion of a receptacle housing 151 installed at an outside of the water level sensing mounting part 121 of the cover 120, a plurality of electrodes 153a, 153b, and 153c extending downwards from the receptacle housing 151, and a plurality of terminals 152a, 152b, and 152c formed at an upper portion of the receptacle housing 151 and connected to a controller (not shown) and respective electrodes 153a, 153b, and 153c.

Here, the number of the electrodes 153a, 153b, and 153c is not limited to the above-described case, and may be determined in accordance with the number of water levels to be measured. In addition, the electrodes 153a, 153b, and 153c are installed at appropriate heights from a bottom surface of the housing 110 in order to sense a level of steam water to be stored in the steam generation device 100. In the present disclosure, three electrodes, that is, a low water level electrode 153a, a first water level electrode 153b, and a second water level electrode 153c may be provided.

Meanwhile, each of the low water level electrode 153a, the first water level electrode 153b, and the second water level electrode 153c is provided to sense a water level in accordance with electrical connection thereof to the common electrode 170. Accordingly, the common electrode 170 may extend to the inside of the heating space by a length equal to or greater than the length of the low water level electrode 153a configured to sense a lowest water level among the low water level electrode 153a, the first water level electrode 153b, and the second water level electrode 153c.

Here, the low water level electrode 153a is an electrode configured to sense a lowest water level at which the first heater 130 disposed at the lower portion of the housing 110 operates stably. The low water level electrode 153a as described above may extend to the lower portion of the heating space, corresponding to a safe operation water level of the first heater 130.

In addition, the first water level electrode 153b is configured to sense a water level of steam water to be heated by the first heater 130. The first water level electrode 153b may be disposed at a higher position than that of the low water level electrode 153a. If necessary, the function of the first water level electrode 153b may be carried out through the function of the low water level electrode 153a.

In addition, the first water level electrode 153b may be provided to sense a water level at a position equal to or lower than that of the lower barrier wall 180. When the first water level electrode 153b senses a water level higher than the lower barrier wall 180, steam is generated from steam water by the first heater 130 at a water level of steam water higher than the lower barrier wall 180. In this case, steam generated by the first heater 130 cannot pass through the through holes 181 of the lower barrier wall 180 and, as such, diffusivity of steam may be degraded. When it is unnecessary to take diffusivity of steam into consideration, the first water level electrode 153b may be disposed at a position higher than the lower barrier wall 180.

Meanwhile, the second water level electrode 153c is provided to sense a water level of steam water when the steam water is heated by the second heater 140. Accordingly, the second water level electrode 153c may extend from the cover 120 to the inside of the heating space in order to sense a water level higher than the second heater 140.

That is, the second heater 140 may generate steam by re-heating steam or heating steam water in accordance with a water level of supplied steam water. For example, for generation of saturated steam, the second heater 140 may re-heat steam generated through steam water heating of the first heater 130 in accordance with water level sensing of the first water level electrode 153b.

Otherwise, in accordance with water level sensing of the second water level electrode 153c, the first heater 130 and the second heater 140 may simultaneously heat steam water, thereby more rapidly generating steam or generating an increased amount of steam.

The temperature sensor 160 is installed at the temperature sensor mounting part 122 of the cover 120 to sense an internal temperature of the heating space. The temperature sensor 169 as described above senses a temperature of steam generated in the heating space, thereby sensing generation of steam or overheated steam.

In this case, the temperature sensor 160 may further include a temperature sensor bracket 161 configured to couple the temperature sensor 160 to the temperature sensor mounting part 122 of the cover 120. Accordingly, the temperature sensor 160 may be coupled to the temperature sensor mounting part 122 of the cover 120 by the temperature sensor bracket 161, and may extend to the inside of the heating space while extending through the cover 120. It is preferred that the temperature sensor 160 as described above be disposed at a relatively upper position in the heating space because the temperature sensor 160 should sense a temperature of steam in the heating space.

The common electrode 170 is configured to be electrically connected to the low water level electrode 153a, the first water level electrode 153b, and the second water level electrode 153c of the water level sensing unit 150. The common electrode 170 may extend by a length equal to or greater than that of the low water level electrode 153a such that the common electrode 170 extends to the inside of the heating space.

In this case, the common electrode 170 may further include a common electrode bracket 171 configured to couple the common electrode 170 to the common electrode mounting part 123 of the cover 120. Accordingly, the common electrode 170 may be coupled to the common electrode mounting part 123 of the cover 120 by the common electrode bracket 171, and may extend to the interior of the heating space while extending through the cover 120.

Hereinafter, operation of the clothing treatment apparatus according to the embodiment of the present disclosure will be described in detail in conjunction with embodiments. Constituent elements, which will be described hereinafter, should be understood with reference to the following detailed description and the accompanying drawings.

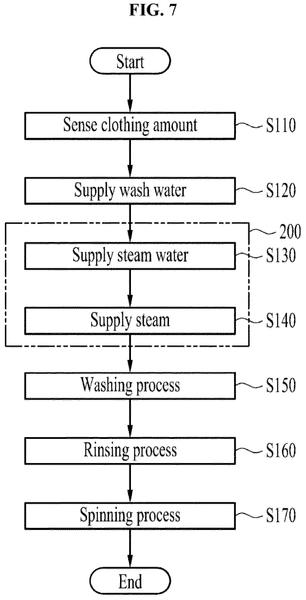

FIG. 7 is a flowchart showing an operation procedure of a clothing treatment apparatus including the steam generation device according to the embodiment of the present disclosure.

In the present disclosure, operation of the clothing treatment apparatus will be described in conjunction with an example in which the clothing treatment apparatus is a washing machine. However, the present disclosure may also be applied to other clothing treatment apparatuses using the steam generation device of the present disclosure. For example, it should be understood that the case in which the present disclosure is applied to a drying device configured to dry an object to be washed using steam or a clothing management device configured to manage clothing using steam falls in the category of the present disclosure.

General washing machines may perform a washing process, a rinsing process, a spinning process, and a drying process. Procedures of the washing process, the rinsing process, the spinning process, and the drying process as described above are identical to general procedures and, as such, no detailed description thereof will be given.

First, when preparation of washing is completed as clothing is loaded in an object accommodation part such as a drum in accordance with start of operation of the clothing treatment apparatus, one of a plurality of washing courses is selected, and the selected washing course is begun.

After starting of the washing course, an amount of clothing to be washed, that is, a clothing amount, is sensed (S110). Based on the clothing amount, wash water is supplied to a tub or a drum (S120). Clothing wetting is performed simultaneously with or after supply of wash water for a predetermined time.

After completion of clothing wetting, post-washing or main washing S150 is performed. After main washing, drainage of wash water is carried out and, as such, a washing process is completed. After completion of the washing process, a rinsing process S160 and a spinning process S170 are sequentially performed and, as such, the washing course is completed.

The washing course has been described in conjunction with an example of a general washing course in which washing is performed using only wash water. During execution of the washing course, a steaming process S200 may be additionally performed.

In more detail, the steaming process S200 may be performed between the water supply procedure S120 and the washing process S150. That is, the steaming process S200 may be performed during execution of clothing wetting. That is, the steaming process S200 may be performed before execution of the washing process S150 performed under the condition that washing water is no longer added.

The steaming process S200 is a course for performing washing using wash water and steam. Accordingly, steam water may be supplied to the steam generation device 100 after the water supply procedure S120. Meanwhile, in the steaming process S200, the amount and temperature of steam supplied from the steam generation device 100 may be varied in accordance with a clothing amount or a clothing quality.

For example, the steaming process S200 may be performed in order to supply steam until an internal temperature of the drum reaches a predetermined temperature as steam is supplied to an interior of the drum. Of course, the steaming process S200 may be performed for a predetermined time. The steam generation device 100 may control an amount of steam generated from the steam generation device 100 by controlling a water level of steam water supplied to the steam generation device 100 and the first and second heaters 130 and 140 in accordance with a predetermined temperature.

In another example, the steaming process S200 may be performed such that steam is supplied to the interior of the drum to heat clothing loaded in the interior of the drum to a predetermined temperature or more, thereby sterilizing the clothing. The amount of steam in this case may be increased or decreased in accordance with the amount of the clothing. The steam generation device 100 may control an amount of steam generated from the steam generation device 100 by controlling a water level of the steam water supplied to the steam generation device 100 and the first and second heaters 130 and 140 in accordance with the sensed clothing amount.

The steam generation device 100 as described above may repeatedly perform control of supply of the steam water and control of the first and second heaters 130 and 140 in any case in which steam is supplied.

Hereinafter, operation of the steam generation device 100 associated with the above-described operation of clothing treatment apparatus will be described in detail. The operation of the steam generation device 100 according to the present disclosure may be divided into different procedures in accordance with an amount and a temperature of supplied steam and, as such, will be described in conjunction with different steam supply procedures.

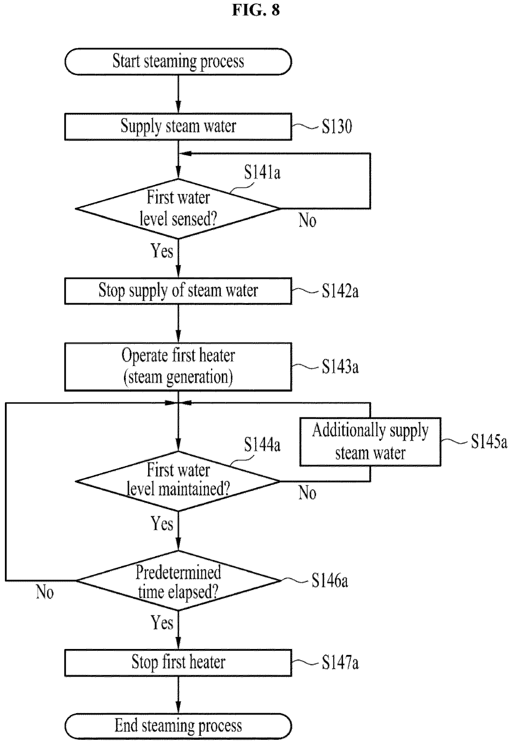

First, a normal steam generation procedure of the clothing treatment apparatus of the present disclosure will be described. FIG. 8 is a flowchart showing a steam generation procedure of the steam generation device according to an embodiment of the present disclosure.

As shown in the drawing, in the clothing treatment apparatus of the present disclosure, steam water is supplied to the steam generation device 100, for supply of steam (S130). As the steam water is supplied, the water level sensing unit 150 may determine a water level of the steam water supplied to the heating space through the low water level electrode 153a and the first water level electrode 153b (S141a).

In this case, when it is determined by the first water level electrode 153b of the water level sensing unit 150 that the water level of the steam water supplied to the heating space reaches a first water level, supply of the steam water may be stopped (S142a). As the steam water reaches the first water level, electric power is then supplied to the first heater 130, and the steam water reaching the first water level is heated by a heating operation of the first heater coil 131, thereby generating steam (S143a).

Meanwhile, when it is sensed by the low water level electrode 153a that the water level of the steam water supplied to the heating space is a low water level, the first heater 130 starts to operate and, as such, preheating of the steam water may be performed before the steam water reaches the first water level. In this case, it may be possible to generate steam within a shorter time than that of the case in which steam is generated through heating of the steam water after the steam water reaches the first water level.

Thereafter, the water level sensing unit 150 senses a variation in water level of the steam water according to generation of steam by the first heater 130 (S144a). In this case, when it is sensed by the first water level electrode 153b that the water level of the steam water is lowered below the first water level, steam water may be additionally supplied for supplement of steam water (S145a).

Meanwhile, when steam is generated in accordance with application of electric power to the first heater 130, whether or not a predetermined time according to generation of steam has elapsed is determined (S146a). When the predetermined time has not elapsed, the first water level sensing S144a and the additional steam water supply S145a may be repeatedly performed.

In this case, the steam generated through the above-described procedure is supplied to the accommodation part through the steam discharge port 120b provided at the cover 120, and the operation of the first heater 130 is stopped when the predetermined time according to generation of steam elapses and, as such, a steaming process may be ended (147a).

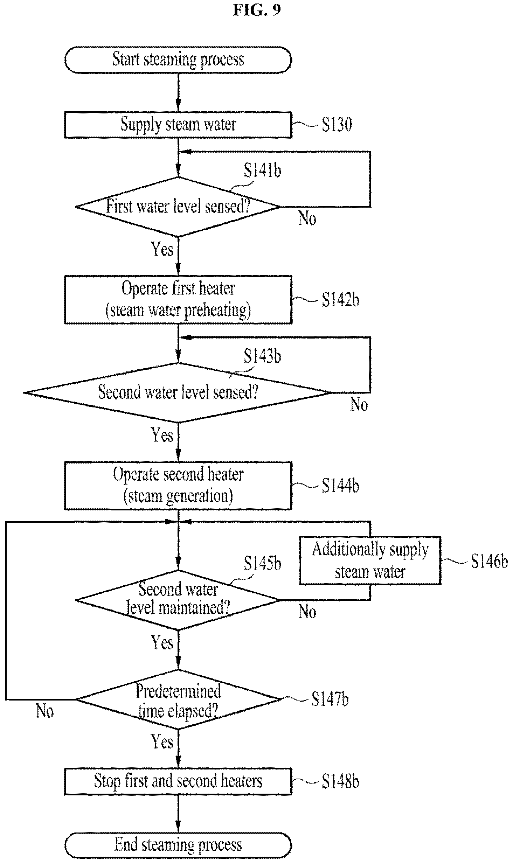

Meanwhile, in the clothing treatment apparatus of the present disclosure, a large amount of steam may be required in accordance with an amount of clothing. In association with this case, a procedure for supplying a large amount of steam will be described in detail. FIG. 9 is a flowchart showing a procedure for generating a large amount of steam in the steam generation device in accordance with an embodiment of the present disclosure.

As shown in the drawing, in the clothing treatment apparatus of the present disclosure, steam water is supplied to the steam generation device 100, for supply of steam (S130). As the steam water is supplied, the water level sensing unit 150 may determine a water level of the steam water supplied to the heating space through the low water level electrode 153a and the first water level electrode 153b (S141b). In this case, when it is determined by the first water level electrode 153b of the water level sensing unit 150 that the water level of the steam water supplied to the heating space reaches the first water level, supply of the steam water may be stopped.

As the steam water reaches the first water level, electric power is then supplied to the first heater 130, and the steam water reaching the first water level may be preheated by a heating operation of the first heater coil 131 (S142b). That is, the water level of the steam water reaching the first water level is a medium water level before the steam water reaches a second water level. Accordingly, it may be possible to preheat the steam water supplied to the firs water level by supplying the electric power to the first heater 130 before the steam water is supplied up to the second water level and, as such, to reduce the heating time of the steam water heated by the second heater 140 at the second water level

Meanwhile, the water level sensing unit 150 determines whether or not the water level of the steam water supplied over the first water level has reached the second water level (S143b). In this case, when the water level of the steam water reaches the second water level, electric power is supplied to the second heater 140, and the steam water reaching the second water level is heated by the second heater coil 141, thereby generating steam.

In this case, the first heater coil 131 of the first heater 130 heats the steam water below the first water level, and the second heater coil 141 of the second heater 140 heats the steam water between the first water level and the second water level. Accordingly, the entirety of the steam water in the heating space is rapidly heated, thereby generating steam.

In addition, the first heater coil 131 of the first heater 130 preheats steam water as the steam water is supplied up to the first water level, and the second heater coil 141 of the second heater 140 heats steam water after the steam water is supplied up to the second water level. Accordingly, it may be possible to generate steam through preheating and heating of steam water at earlier times than a time at which the first heater 130 and the second heater 140 operate after the steam water is completely supplied up to the second water level.

Thereafter, the water level sensing unit 150 senses a variation in water level of the steam water according to generation of steam by the first heater 130 (S145b). In this case, when it is sensed by the second water level electrode 153c that the water level of the steam water is lowered below the second water level, steam water may be additionally supplied for supplement of steam water (S146b).

Meanwhile, when steam is generated in accordance with application of electric power to the second heater 140, whether or not a predetermined time according to generation of steam has elapsed is determined (S147b). When the predetermined time has not elapsed, the second water level sensing S145b and the additional steam water supply S146b may be repeatedly performed.

In this case, the steam generated through the above-described procedure is supplied to the accommodation part through the steam discharge port 120b provided at the cover 120, and the operations of the first heater 130 and the second heater 140 are stopped when the predetermined time according to generation of steam elapses and, as such, a steaming process may be ended (S148b).

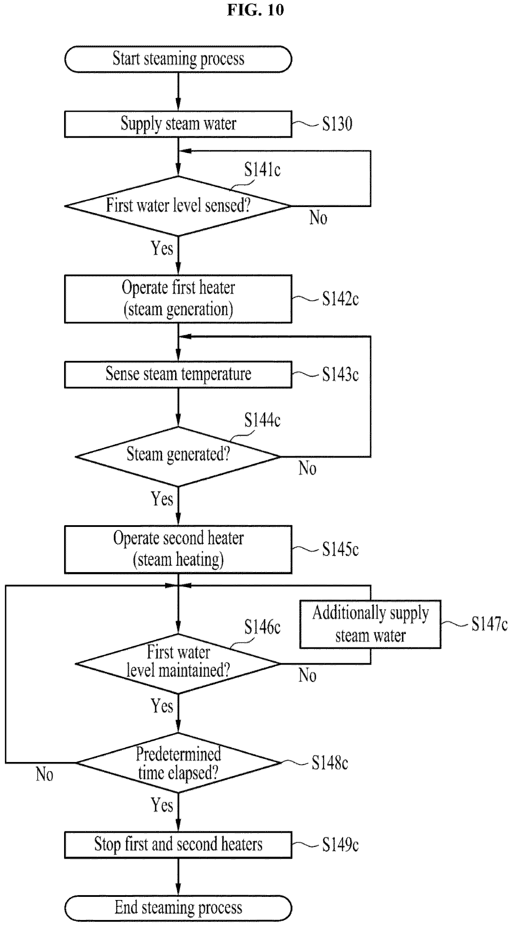

Meanwhile, in the clothing treatment apparatus of the present disclosure, steam of a relatively high temperature may be required as a washing process proceeds. In association with this case, a procedure for supplying steam of a high temperature will be described in detail. FIG. 10 is a flowchart showing a procedure for generating overheated steam in the steam generation device in accordance with an embodiment of the present disclosure.

As shown in the drawing, in the clothing treatment apparatus of the present disclosure, steam water is supplied to the steam generation device 100, for supply of steam (S130). As the steam water is supplied, the water level sensing unit 150 may determine a water level of the steam water supplied to the heating space through the low water level electrode 153a and the first water level electrode 153b (S141c). In this case, when it is determined by the first water level electrode 153b of the water level sensing unit 150 that the water level of the steam water supplied to the heating space reaches the first water level, supply of the steam water may be stopped.

As the steam water reaches the first water level, electric power is then supplied to the first heater 130, and the steam water reaching the first water level is heated by a heating operation of the first heater coil 131, thereby generating steam (S142a).

Meanwhile, the temperature sensor 160 may determine whether or not steam is generated, by sensing a temperature of steam generated as the steam water is heated at the first water level by the first heater 130 (S143c). When the temperature sensed by the temperature sensor 160 does not reach a steam generation temperature, it is determined that steam has not been generated, and subsequent control waits until the sensed temperature reaches the steam generation temperature (S144c).

In this case, the steam generated at the first water level by the first heater 130 may not only move between the lower barrier wall 180 and the upper barrier wall 190 through the through holes 181 of the lower barrier wall 180, but also may move between the lower barrier wall 180 and the upper barrier wall 190 through the space between the lower barrier wall 180 and the side wall 112a of the housing 110.

Meanwhile, when the steam generation temperature is sensed by the temperature sensor 160, electric power is supplied to the second heater 140. In this case, the steam present between the lower barrier wall 180 and the upper barrier wall 190 is re-heated in accordance with a heating operation of the second heater coil 141 of the second heater 140 and, as such, is changed into overheated steam (S145c).

In this case, the overheated steam generated between the lower barrier wall 180 and the upper barrier wall 190 through heating by the second heater 140 has a higher temperature/higher pressure than those of the steam generated by the first heater 130. Accordingly, the overheated steam may be more effectively used for heating of wash water, heating of clothing, and sterilization of clothing.

In this case, the overheated steam generated between the lower barrier wall 180 and the upper barrier wall 190 through heating moves to an upper side of the upper barrier wall 190 through the through holes 191 of the upper barrier wall 190 and the space formed between the upper barrier wall 190 and the side wall 112c of the housing 110.

Thereafter, the water level sensing unit 150 senses a variation in water level of the steam water according to generation of steam by the first heater 130 (S146c). In this case, when it is sensed by the first water level electrode 153b of the water level sensing unit 150 that the water level of the steam water is lowered below the first water level, steam water may be additionally supplied for supplement of steam water (S147c).

Meanwhile, when steam is generated in accordance with application of electric power to the first heater 130, whether or not a predetermined time according to generation of steam has elapsed is determined (S148c). When the predetermined time has not elapsed, the first water level sensing S146c and the additional steam water supply S147c may be repeatedly performed.

In this case, the steam generated through the above-described procedure is supplied to the accommodation part through the steam discharge port 120b provided at the cover 120, and the operations of the first heater 130 and the second heater 140 are stopped when the predetermined time according to generation of steam elapses and, as such, a steaming process may be ended (147a).

Although the preferred embodiments of the present disclosure have been disclosed for illustrative purposes, those skilled in the art will appreciate that various modifications are possible, without departing from the scope and spirit of the disclosure as disclosed in the accompanying claims. Accordingly, it is to be appreciated that all modifications of embodiments of the present disclosure, which will be made, are encompassed in the present disclosure.

## Claims

1. A clothing treatment apparatus comprising a cabinet, an accommodation part disposed inside the cabinet and configured to accommodate an object, and a steam generation device disposed outside the object accommodation part and configured to generate steam to be supplied to the object accommodation part, wherein the steam generation device comprises:
a housing configured to receive steam water and to form a heating space;
a cover configured to cover the heating space;
a first heater configured to preheat the steam water at a first water level or to heat the steam water, thereby generating steam; and
a second heater configured to heat steam water at a second water level higher than the first water level, thereby generating steam, or to heat the steam generated at the first water level in a space between the first water level and the second water level, thereby generating overheated steam.

2. The clothing treatment of claim 1, wherein the steam generation device further comprises a water level sensing unit configured to sense the first water level and the second water level of steam water supplied to the heating space.

3. The clothing treatment apparatus of claim 2, wherein the water level sensing unit comprises a first water level electrode configured to sense the first water level, and a second water level electrode configured to sense the second water level.

4. The clothing treatment apparatus of claim 3, wherein the first heater preheats and heats the steam water of the first water level in accordance with water level sensing of the first water level electrode and the second heater heats the steam water of the second water level in accordance with water level sensing of the second water level electrode, thereby generating steam.

5. The clothing treatment apparatus of claim 1, wherein:
the steam generation device further comprises a temperature sensor configured to sense an internal temperature of the heating space, thereby sensing generation of steam; and
the second heater heats steam over the first water level in accordance with steam sensing of the temperature sensor, thereby generating overheated steam.

6. The clothing treatment apparatus of claim 1, wherein the steam generation device further comprises:
a lower barrier wall configured to form a movement path of the steam generated at the first water level; and
an upper barrier wall disposed over the second heater and configured to form a movement path of the steam together with the lower barrier wall.

7. The clothing treatment apparatus of claim 6, wherein the second heater is disposed between the lower barrier wall and the upper barrier wall and heats the steam moving between the lower barrier wall and the upper barrier wall, thereby generating overheated steam.

8. The clothing treatment apparatus of claim 6, wherein each of the lower barrier wall and the upper barrier wall is formed with a plurality of through holes configured to diffuse the steam and the overheated steam.

9. The clothing treatment apparatus of claim 6, wherein the lower barrier wall is provided at the housing such that one side thereof is opened, and the upper barrier wall is provided at the housing such that another side thereof is opened, to form the movement path of the steam generated at the first water level.

10. A clothing treatment apparatus comprising a cabinet, an accommodation part disposed inside the cabinet and configured to accommodate an object, and a steam generation device disposed outside the object accommodation part and configured to generate steam to be supplied to the object accommodation part, wherein the steam generation device comprises:
a housing configured to receive steam water and to form a heating space;
a cover configured to cover the heating space;
a water level sensing unit configured to sense a first water level and a second water level of steam water supplied to the heating space, the second water level being higher than the first water level;
a first heater configured to heat steam water below the first water level; and
a second heater configured to heat steam generated over the first water level through heating by the first heater, thereby generating overheated steam, or to heat the steam water supplied up to the second water level, thereby generating steam.

11. The clothing treatment apparatus of claim 10, wherein the water level sensing unit comprises a first water level electrode configured to sense the first water level, and a second water level electrode configured to sense the second water level.

12. The clothing treatment apparatus of claim 11, wherein the first heater preheats and heats the steam water of the first water level in accordance with water level sensing of the first water level electrode and the second heater heats the steam water of the second water level in accordance with water level sensing of the second water level electrode, thereby generating steam.

13. The clothing treatment apparatus of claim 10, wherein:
the steam generation device further comprises a temperature sensor configured to sense an internal temperature of the heating space, thereby sensing generation of steam; and
the second heater heats steam over the first water level in accordance with steam sensing of the temperature sensor, thereby generating overheated steam.

14. The clothing treatment apparatus of claim 10, wherein the steam generation device further comprises:
a lower barrier wall configured to form a movement path of the steam generated at the first water level; and
an upper barrier wall disposed over the second heater and configured to form a movement path of the steam together with the lower barrier wall.

15. The clothing treatment apparatus of claim 14, wherein the second heater is disposed between the lower barrier wall and the upper barrier wall and heats the steam moving between the lower barrier wall and the upper barrier wall, thereby generating overheated steam.

16. The clothing treatment apparatus of claim 14, wherein each of the lower barrier wall and the upper barrier wall is formed with a plurality of through holes configured to diffuse the steam and the overheated steam.

17. The clothing treatment apparatus of claim 14, wherein the lower barrier wall is provided at the housing such that one side thereof is opened, and the upper barrier wall is provided at the housing such that another side thereof is opened, to form the movement path of the steam generated at the first water level.

18. A clothing treatment apparatus comprising a cabinet, an accommodation part disposed inside the cabinet and configured to accommodate an object, and a steam generation device disposed outside the object accommodation part and configured to generate steam to be supplied to the object accommodation part, wherein:
the steam generation device comprises:
a box-shaped housing configured to receive steam water supplied thereto;
a cover configured to cover the heating space;
a first heater disposed under the housing;
a lower barrier wall disposed over the first heater;
a second heater disposed over the lower barrier wall; and
an upper barrier wall disposed over the second heater; and
steam generated through heating by the first heater is moved to a space between the lower barrier wall and the upper barrier wall and is then changed into overheated steam by the second heater.

19. The clothing treatment apparatus according to claim 18, wherein the lower barrier wall guides the steam generated by the first heater to move between the lower barrier wall and the upper barrier wall, and the lower barrier wall and the upper barrier wall form a steam heating space heated by the second heater.

20. The clothing treatment apparatus of claim 18, wherein each of the lower barrier wall and the upper barrier wall is formed with a plurality of through holes configured to diffuse the steam and the overheated steam.

21. The clothing treatment apparatus of claim 18, wherein the lower barrier wall is provided at the housing such that one side thereof is opened, and the upper barrier wall is provided at the housing such that another side thereof is opened, to form a movement path of the steam generated at the first water level.

22. A clothing treatment apparatus comprising a cabinet, an accommodation part disposed inside the cabinet and configured to accommodate an object, and a steam generation device disposed outside the object accommodation part and configured to generate steam to be supplied to the object accommodation part, wherein:
the steam generation device comprises:
a housing configured to receive steam water and to form a heating space;
a cover configured to cover the heating space;
a water level sensing unit configured to sense a first water level and a second water level of steam water supplied to the heating space, the second water level being higher than the first water level;
a first heater disposed below the first water level;
a second heater disposed between the first water level and the second water level;
a lower barrier wall disposed between the first water level and the second heater; and
an upper barrier wall disposed above the second water level and configured to form a steam flow path together with the lower barrier wall; and
the second heater heats steam generated through heating by the first heater when the steam water is disposed at the first water level, and heats the steam water together with the first heater when the steam water is disposed at the second water level, thereby generating steam.

23. The clothing treatment apparatus of claim 22, wherein:
the water level sensing unit comprises a first water level electrode configured to sense the first water level, and a second water level electrode configured to sense the second water level; and
the first heater preheats and heats the steam water of the first water level in accordance with water level sensing of the first water level electrode.

24. The clothing treatment apparatus of claim 22, wherein:
the steam generation device further comprises a temperature sensor configured to sense an internal temperature of the heating space, thereby sensing generation of steam; and
the second heater heats steam over the first water level in accordance with steam sensing of the temperature sensor, thereby generating overheated steam.

25. A control method of a clothing treatment apparatus comprising a steam generation device comprising a water level sensing unit configured to sense a first water level and a second water level of steam water supplied to a heating space, the second water level being higher than the first water level, a first heater disposed below the first water level, and a second heater disposed between the first water level and the second water level, the control method comprising:
water supply of supplying steam water to the steam generation device;
first water level sensing of sensing the first water level of the supplied steam water;
preheating of preheating the steam water of the first water level by the first heater;
second water level sensing of sensing the second water level higher than the first water level; and
heating of heating the steam water of the second water level by the second heater in accordance with the sensing of the second water level, thereby generating steam.

26. The control method of claim 25, further comprising:
second water level maintenance sensing of determining whether or not a water level of the steam water is maintained at the second water level, after the heating.

27. The control method of claim 26, further comprising:
additional water supply of supplying additional steam water when the water level of the steam water is lowered below the second water level in the second water level maintenance sensing.

28. A control method of a clothing treatment apparatus comprising a steam generation device comprising a water level sensing unit configured to sense a first water level and a second water level of steam water supplied to a heating space, the second water level being higher than the first water level, a first heater disposed below the first water level, and a second heater disposed between the first water level and the second water level, the control method comprising:
water supply of supplying steam water to the steam generation device;
first water level sensing of sensing the first water level of the supplied steam water;
heating of heating the steam water of the first water level by the first heater, thereby generating steam; and
overheated steam generation of heating the steam generated at the first water level by the second heater, thereby generating overheated steam.

29. The control method of claim 28, further comprising:
steam sensing of measuring a temperature of the heating space after the heating, thereby sensing generation of the steam.

30. The control method of claim 29, further comprising:
first water level maintenance sensing of determining whether or not a water level of the steam water is maintained at the first water level, after the heating.

31. The control method of claim 30, further comprising:
additional water supply of supplying additional steam water when the water level of the steam water is lowered below the second water level in the first water level maintenance sensing.
